# EUROPEAN PATENT APPLICATION

(11) **EP 4 831 208 A1**
(43) Date of publication of application: **28.10.2026**
(21) Application number: 23961799.6
(22) Date of filing: 19.12.2023
(51) Int. Cl.: C07K 19/00, C12N 15/62, A61K 38/20, A61K 39/395, A61P 35/00

(54) **BIFUNCTIONAL FUSION PROTEIN COMPOSED OF IL-15 AND T CELL CO-STIMULATORY MOLECULE ANTIBODY**

(71) Applicant: ImmuneLogic Therapeutics, Inc., Beijing 102206 (CN)
(72) Inventor: FU, Yangxin, Beijing 100101 (CN); PENG, Hua, Beijing 100101 (CN); CAI, Yueqi, Beijing 100101 (CN)
(74) Representative: Lavoix
(86) International application number: PCT/CN2023/139925
(87) International publication number: WO 2025/129454

(57) **Abstract**

Provided is a bifunctional fusion protein composed of IL-15 and a T cell co-stimulatory molecule antibody. The T cell co-stimulatory molecule is 4-1BB, ICOS or OX40, preferably 4-1BB. The fusion protein comprises: (1) a T cell co-stimulatory molecule antibody; (2) a conjugate of IL-15 and IL-15R Sushi domain, wherein the IL-15 and IL-15R Sushi domain are linked by means of a second linker fragment; and (3) a first linker fragment, wherein the first linker fragment is used for linking a heavy chain Fc region of the T cell co-stimulatory molecule antibody and the conjugate of IL-15 and IL-15R Sushi domain, and can be cleaved by matrix metalloproteinase.

## Description

### TECHNICAL FIELD

The present disclosure belongs to the technical field of biomedicine, and specifically, relates to a bifunctional fusion protein composed of IL-15 and an antibody against a T cell co-stimulatory molecule.

### BACKGROUND

Interleukin-15 (IL-15) is a 4 alpha-helical glycoprotein composed of 114 amino acids and has a molecular weight of 14-15 kDa [1]. IL-15 is mainly secreted by dendritic cells (DCs), macrophages and monocytes and acts on other cells. Receptors for IL-15 include IL-15Rα (CD215), IL-2Rβ (CD122) and IL-2Rγ (CD132). CD215 is mainly expressed on the surface of DCs, macrophages and monocytes, and IL-15, after secreted by cells, can directly bind to CD215 and be trans-presented to T cells and NK cells expressing IL-2Rβ and IL-2Rγ. After binding to the receptors, IL-15 can phosphorylate downstream STAT3 and STAT5 and activate JAK1 and JAK3 signal pathways [2-4].

IL-15 mainly promotes the proliferation and activation of T cells and NK cells and maintains the homeostasis of lymphocytes. IL-15 can also maintain the survival of memory CD8⁺T cells and effector T cells and prevent T cells from producing activation-induced cell death (AICD) [5]. IL-15 does not bind to IL-2Rα and does not induce the proliferation of regulatory T cells (Tregs).

In tumor therapy, IL-15 monomer is greatly limited in clinical use due to its short half-life and weak activity. Because IL-15 needs to bind to IL-15Rα for stronger affinity, the complex form of IL-15 and IL-15Rα has become the main use form in the current clinical research. In addition, N72D, an IL-15 mutant, can further enhance the activity of IL-15 [6]. By enhancing IL-15' half-life and activity, the anti-tumor activity of IL-15 can be improved to a certain extent, but the anti-tumor effect is extremely weak when it is used as monotherapy. Therefore, IL-15 is currently more used in combination therapies with other therapies, such as PD-1 antibody, CTLA-4 antibody, CD20 antibody and CD52 antibody. Because NK cells in peripheral tissues express more IL-2Rβ, IL-15 preferentially binds to peripheral NK cells. These forms of IL-15 are not selective for proliferation and activation of NK cells and T cells, causing significant proliferation of peripheral T cells, especially NK cells, and the enhancement of its activity and half-life also brings an increase in side effects. Many studies have shown that the toxicity caused by IL-15 is mainly caused by peripheral NK cells [7].

To solve the problem of peripheral toxicity caused by IL-15, prior studies mainly focus on fusion of IL-15 with other antibodies or polypeptides, in order to change the targeting of the fusion protein, reduce the peripheral binding of IL-15, increase IL-15's binding in tumor cells, enrich the fusion protein in tumors and allow IL-15 to play an activation role on immune cells. For example, by fusing with IL-15/IL-15Rα, the RGD polypeptide can specifically bind to integrin αvβ3, which is highly expressed in tumor cells and endothelial cells, so that the fusion protein can be enriched in tumors, which allows the drug concentration in tumors to increase and allows IL-15/IL-15Rα to exert the activity [8]. Given that the affinity between IL-15 and IL-15Rα is about 38 pM and the affinity between IL-15 and CD122/CD132 also reaches 1 nM [9, 10], which is close to that between IL-15 and antibody (10⁻⁸ ~ 10⁻¹⁰ M), the fusion protein obtained by fusing IL-15 with antibody has targeting, which cannot be determined by antibody, and thus the affinity between IL-15 and its receptor needs to be reduced so as to better target the fusion protein into tumors. Some studies show that IL-15 can directly act on intratumoral T cells and exert its anti-tumor effect by mutating IL-15 to reduce its affinity with the receptors IL-15Rα and CD122/CD132, fusing the IL-15 with a screened PD-1 antibody with high affinity, and targeting the resultant fusion protein into T cells with high PD-1 expression in tumors [11]. In addition to altering the targeting of the fusion protein by mutating IL-15 to reduce affinity, IL-15 can be blocked by linking IL-15 to the extracellular domain of receptor IL-2Rβ through a matrix metalloproteinase-14 (MMP-14)-recognizable substrate sequence, and when the MMP-14-recognizable sequence is cleaved in tumors, IL-15 is released and activates the anti-tumor immune response [12].

With continuous improvement, IL-15 can effectively expand intratumoral CD8+T cells and NK cells, and improve anti-tumor response. However, tumors often exhibit an immunosuppressive state, and thus the expanded immune cells quickly become in an exhausted state or the activity thereof is suppressed by intratumoral immunosuppressive cells, and thus cannot exert the anti-tumor effect. Immunosuppressive cells in the tumor microenvironment (TME) mainly include tumor associated macrophages (TAMs), myeloid-derived suppressor cells (MDSCs) and Tregs, etc. [13]. How to effectively inhibit immunosuppressive effect of these cells has become a key problem to be solved in the immunotherapy. The intratumoral Tregs inhibit anti-tumor immune response in the following ways: 1) highly expressing CTLA-4 to compete with CD80 and CD86 expressed by DCs; 2) highly expressing CD25 to compete with effector T cells for IL-2; 3) secreting inhibitory cytokines such as TGF-β and IL-10, etc. to inhibit the function of effector T cells; 4) regulating the metabolism level of tryptophan and adenosine to inhibit the function of effector T cells [14]. An increase in the proportion of intratumoral Tregs/CD8+T cells is often associated with poor prognosis. An increase in the number of Tregs is detrimental to the anti-tumor immune response in most cancer types, although it is not the case in some cancer types. Therefore, the effective depletion of intratumoral Tregs without affecting peripheral Tregs can effectively enhance the anti-tumor immune response.

Tregs in the tumor microenvironment often highly express CD25, CTLA-4, ICOS, OX40, CCR4 and CCR8, and antibodies against these molecules are often used to deplete Tregs. Some of these molecules, such as CD25 and CTLA-4, which are highly expressed in intratumoral Tregs, are also transiently highly expressed in effector T cells, and thus when the corresponding antibodies are used to deplete Tregs, effector T cells will be depleted too, which results in a great decrease in anti-tumor effect. Selective depletion of intratumoral Tregs with an antibody against an appropriate marker molecule becomes the key to breaking Tregs-induced immune tolerance.

ICOS, OX40, GITR and 4-1BB, etc. in the tumor necrosis factor receptor superfamily (TNFRSF) members are often constitutively expressed in Tregs and up-regulated in tumors. These molecules are of increasing interest. Initially, antibodies against these targets are often used to activate co-stimulatory molecules to activate T cells. Although some studies have shown that antibodies against these molecules can be used to deplete Tregs, there is no clinical trial for Tregs depletion yet.

4-1BB molecule is constitutively expressed in Tregs and DCs and inducibly expressed in T cells and NK cells. In T cells, TCR stimulation or CD3 signal can induce the upregulation of 4-1BB expression, which, together with TCR signal, synergistically promote the secretion of IL-2 and IFN-γ in T cells and the proliferation of T cells [15]. Further studies have shown that 4-1BB co-stimulation can not only induce effector cells to produce effector factors, but also facilitate the differentiation of memory T cells and effector cells and protect T cells from apoptosis [16]. In tumors, 4-1BB molecule is up-regulated in both T cells and NK cells, especially in Tregs. The function of 4-1BB molecule in Th1 CD4⁺T cells and Tregs remains unclear. Activation of 4-1BB signal helps to activate NK cells and enhance ADCC and cytotoxic effect thereof [17]. In addition, the high expression of 4-1BB molecule in Tregs makes this molecule an important target for effectively reducing and depleting the intratumoral Tregs, which provides an auxiliary means for immunotherapy. In a mouse model, the use of 4-1BB depletion-type antibody can effectively inhibit tumor growth and deplete intratumoral Tregs, and its effect of depleting Tregs is equivalent to that of CTLA-4 and OX40 antibodies. Meanwhile, after the therapy with 4-1BB antibody, Tregs cells which are not depleted in tumors exhibit a weak inhibitory phenotype [18]. Although some CD8⁺T cells in tumors also express 4-1BB and the use of 4-1BB antibody may affect the number of this portion of T cells while depleting Tregs, multiple studies have shown that, in a mouse tumor model, the IgG1 isotype 4-1BB antibody has a far less anti-tumor effect than the IgG2a isotype antibody, indicating that the depletion of Tregs is superior to the activation of T cells using 4-1BB antibody in the generation of anti-tumor immune response [19, 20].

On this basis, the present invention is proposed.

### References:

[1] FEHNIGER, T. A. Interleukin 15: biology and relevance to human disease [J]. 2001, 97(1): 14-32.
[2] WALDMANN, THOMAS A. The biology of interleukin-2 and interleukin-15: implications for cancer therapy and vaccine design [J]. Nature Reviews Immunology, 2006, 6(8): 595-601.
[3] WALDMANN T A, MILJKOVIC M, CONLON K C. Interleukin-15 (dys)regulation of lymphoid homeostasis: Implications for therapy of autoimmunity and cancer [J]. Journal of Experimental Medicine, 2020, 217(1).
[4] FEHNIGER T A, CALIGIURI M A. Fehniger TA, Caligiuri MAInterleukin 15: biology and relevance to human disease. Blood 97:14-32 [J]. Blood, 2001, 97(1): 14-32.
[5] MARKS-KONCZALIK J, DUBOIS S, LOSI J M, et al. IL-2-induced activation-induced cell death is inhibited in IL-15 transgenic mice [J]. Proc Natl Acad Sci U S A, 2000, 97(21): 11445-50.
[6] ZHU X, MARCUS W D, XU W, et al. Novel Human Interleukin-15 Agonists [J]. Journal of Immunology, 2009, 183(6): 3598-607.
[7] GUO Y, LUAN L, RABACAL W, et al. IL-15 Superagonist-Mediated Immunotoxicity: Role of NK Cells and IFN-γ [J]. The Journal of Immunology, 2015: jimmunol.1500300.
[8] CHEN S, HUANG Q, LIU J, et al. A targeted IL-15 fusion protein with potent anti-tumor activity [J]. Cancer Biology & Therapy, 2015, 16(9): 1415-21.
[9] Soluble interleukin-15 receptor alpha (IL-15R alpha)-sushi as a selective and potent agonist of IL-15 action through IL-15R beta/gamma. Hyperagonist IL-15 x IL-15R alpha fusion proteins [J]. Journal of Biological Chemistry, 2006, 281.
[10]GIRI J G, AHDIEH M, EISENMAN J, et al. Utilization of the beta and gamma chains of the ILreceptor by the novel cytokine IL [J]. The EMBO Journal, 1994, 13(12).
[11]An Engineered IL15 Cytokine Mutein Fused to an Anti-PD1 Improves Intratumoral T-cell Function and Antitumor Immunity [J]. Cancer Immunology Research, 2021, 9(10): 1141-57.
[12]Tumor-conditional IL-15 pro-cytokine reactivates anti-tumor immunity with limited toxicity [J]. Cell Research.
[13]VONDERHEIDE, ROBERT H, WEIPING, et al. Understanding the tumor immune microenvironment (TIME) for effective therapy [J].
[14]OHUE Y, NISHIKAWA H. Regulatory T (Treg) cells in cancer: Can Treg cells be a new therapeutic target? [J]. Cancer Science, 2019, 110(7).
[15]MELERO I, BACH N, HELLSTR M K E, et al. Amplification of tumor immunity by gene transfer of the co-stimulatory 4-1BB ligand: synergy with the CD28 co-stimulatory pathway [J]. European Journal of Immunology, 1998.
[16]SHUFORD W W, KLUSSMAN K, TRITCHLER D D, et al. 4-1BB Costimulatory Signals Preferentially Induce CD8+ TCell Proliferation and Lead to the Amplification In Vivo of Cytotoxic T Cell Responses [J]. Journal of Experimental Medicine, 1997, 186(1): 47-55.
[17]KOHRT H E, COLEVAS A D, HOUOT R, et al. Targeting CD137 enhances the efficacy of cetuximab [J]. Journal of Clinical Investigation, 2019, 129(6): 2595-.
[18]MAJ T, WANG W, CRESPO J, et al. Oxidative stress controls regulatory T cell apoptosis and suppressor activity and PD-L1-blockade resistance in tumor [J]. Nature Immunology, 2017.
[19]ASHLEY V, MENK, NICOLE E, et al. 4-1BB costimulation induces T cell mitochondrial function and biogenesis enabling cancer immunotherapeutic responses [J]. The Journal of experimental medicine, 2018.
[20]BUCHAN S L, DOU L, REMER M, et al. Antibodies to Costimulatory Receptor 4-1BB Enhance Anti-tumor Immunity via T Regulatory Cell Depletion and Promotion of CD8 T Cell Effector Function [J]. Immunity, 2018.

### SUMMARY

The present disclosure first relates to a bifunctional fusion protein composed of IL-15 and an antibody against a T cell co-stimulatory molecule;
the T cell co-stimulatory molecule is 4-1BB, ICOS or OX40, preferably 4-1BB;
the fusion protein comprises:
   (1) an antibody against a T cell co-stimulatory molecule;
   (2) a conjugate of IL-15 and IL-15R Sushi domain linked by a second linker; and
   (3) a matrix metalloproteinase-cleavable first linker for linking the Fc region of the heavy chain of the antibody against the T cell co-stimulatory molecule to the conjugate of IL-15 and IL-15R Sushi domain;
preferably, the antibody against the T cell co-stimulatory molecule is an IgG1-type antibody;
preferably, the first linker comprises 2-4 G₄S linking units and a matrix metalloproteinase-recognizable and -cleavable short peptide contained therein;
more preferably, the matrix metalloproteinase-recognizable and -cleavable short peptide has an amino acid sequence as shown in SEQ ID NO.6;
SEQ ID NO.6: SGWLPSSITA;
most preferably, the first linker has an amino acid sequence as shown in SEQ ID NO.12 or SEQ ID NO.11;
SEQ ID NO.12: GGGGSSGWLPSSITAGGGGS;
SEQ ID NO.11: GSSGWLPSSITAGGS;
preferably, the second linker comprises 2-5 GnS linking units, wherein n is an integer of 1-4; more preferably, the second linker has an amino acid sequence as shown in SEQ ID NO.5;
SEQ ID NO.5: GGGGSGGGGSGGGGS.

Further, the bifunctional fusion protein comprises:
(1) a first structural unit composed of a heavy chain of a 4-1BB antibody, the first linker and the conjugate of IL-15 and IL-15R Sushi domain from N-terminus to C-terminus; and
(2) a second structural unit composed of a light chain of the 4-1BB antibody paired with the heavy chain of the 4-1BB antibody;
further, the bifunctional fusion protein is dimerized via the Fc region of the heavy chain of the 4-1BB antibody to form a homodimer.

Preferably,
the 4-1BB antibody is an antibody formed by fusing the Fab region of a human or murine antibody with the Fc region of IgG1,
the heavy chain (VH+CH1) of the murine 4-1BB antibody has an amino acid sequence as shown in SEQ ID NO.2; 
SEQ ID NO.2:
DVQLVESGGGLVQPGRSLKLSCAASGFIFSYFDMAWVRQAPTKGLEWVASISPDGSI
PYYRDSVKGRFTVSRENAKSSLYLQMDSLRSEDTATYYCARRSYGGYSELDYWGQGV
MVTVSSASTKGPSVFPLAPSSKSTSGGTAALGCLVKDYFPEPVTVSWNSGALTSGVHTFP
 AVLQSSGLYSLSSVVTVPSSSLGTQTYICNVNHKPSNTKVDKRV;
the heavy chain (VH+CH1) of the human 4-1BB antibody has an amino acid sequence as shown in SEQ ID NO.10; 
SEQ ID NO.10:
QVQLQESGPGLVKPSETLSLTCTVSGGSISSYYWSWIRQPPGKGLEWIGYIYYSGSTN
YNPSLKSRVTISVDTSKSQFSLKLTSVTAADTAVYYCAKDSDYYGSGSYSYWYFDLWGR
GTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRV;
the light chain (VL-CL) of the murine 4-1BB antibody has an amino acid sequence as shown in SEQ ID NO.1; 
SEQ ID NO.1:
DIQMTQSPASLSASLEEIVTITCQASQDIGNWLAWYHQKPGKSPQLLIYGSTSLADGV
PSRFSGSSSGSQYSLKISRLQVEDIGIYYCLQAYGAPWTFGGGTKLELKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC;
the light chain (VL-CL) of the human 4-1BB antibody has an amino acid sequence as shown in SEQ ID NO.9; 
SEQ ID NO.9:
EIVMTQSPATLSVSPGERATLSCRASQSVSSNLAWYQQKPGQAPRLLIYGASTRAPGI
PARPSGSGSGTEFTLTISSLQSEDFAVYYCQQYNNWPLTFGGGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC;
the Fc region of IgG1 is the Fc region of human IgG1 or a Fc region variant of human IgG1 with ADCC effect knocked out;
the Fc region of human IgG1 has an amino acid sequence as shown in SEQ ID NO.3, and the Fc region variant of human IgG1 with ADCC effect knocked out has an amino acid sequence as shown in SEQ ID NO.4;
   SEQ ID NO.3: 
EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPA
 
PIEKTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK;
   SEQ ID NO.4:

EPKSCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALG
APIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK.

The IL-15 and IL-15R Sushi domain are human or murine proteins,
the murine IL-15 has an amino acid sequence as shown in SEQ ID NO.7, and the murine IL-15R Sushi domain has an amino acid sequence as shown in SEQ ID NO.8;
   SEQ ID NO.7: 
NWIDVRYDLEKIESLIQSIHIDTTLYTDSDFHPSCKVTAMNCFLLELQVILHEYSNMTL
NETVRNVLYLANSTLSSNKNVAESGCKECEELEEKTFTEFLQSFIRIVQMFINTS;
   SEQ ID NO.8: 
LQGTTCPPPVSIEHADIRVKNYSVNSRERYVCNSGFKRKAGTSTLIECVINKNTNVAH
WTTPSLKCIRDPSLAHYSPVPT;
the human IL-15 has an amino acid sequence as shown in SEQ ID NO.13, and the human IL-15R Sushi domain has an amino acid sequence as shown in SEQ ID NO.14;
   SEQ ID NO.13: 
NWVNVISDLKKIEDLIQSMHIDATLYTESDVHPSCKVTAMKCFLLELQVISLESGDAS
IHDTVENLIILANNSLSSNGNVTESGCKECEELEEKNIKEFLQSFVHIVQMFINTS;
   SEQ ID NO.14:

LQITCPPPMSVEHADIWVKSYSLYSRERYICNSGFKRKAGTSSLTECVLNKATNVAH
WTTPSLKCIRDPALVHQRPAPP.

The present disclosure further relates to a nucleotide fragment encoding the bifunctional fusion protein, or a vector or a host comprising the nucleotide fragment.

The present disclosure further relates to use of the bifunctional fusion protein, or the nucleotide fragment encoding the bifunctional fusion protein, or the vector or the host comprising the nucleotide fragment in:
(1) the preparation of an anti-tumor medicament;
(2) the preparation of a combined anti-tumor medicament.

Preferably, the anti-tumor medicament is a medicament that reduces and depletes intratumoral Treg cells and induces proliferation of CD8⁺T cells;
preferably, the tumor is a tumor in an immunosuppressive state;
preferably, the combined anti-tumor medicament is a combined anti-tumor medicament in combination with an immune checkpoint inhibitor; the immune checkpoint inhibitor includes, but is not limited to, a PD-1/PD-L1 antibody, CTLA4 antibody, TIGIT antibody, LAG-3 antibody and TIM-3 antibody.

The present disclosure has the following beneficial effects:
(1) IL-15 has a strong ability to expand CD8+T cells and NK cells, but the expanded T cells do not show a high anti-tumor ability, and the therapeutic effect of IL-15 can be improved by changing the intratumoral immunosuppressive state;
(2) based on the ability of the 4-1BB antibody to deplete intratumoral Tregs, which can relieve inhibition of Tregs on effector T cells, the fusion protein composed of a 4-1BB antibody and IL-15 as bifunctional antibody can simultaneously deplete Tregs and expand CD8+T cells;
(3) by designing a short substrate sequence that can be recognized and cleaved by highly expressed MMP in tumors and is placed between the antibody and IL-15, the short linker peptide can maintain the activity of IL-15 and avoid peripheral toxicity, and the linker peptide can be cleaved by MMP to release IL-15 and restore its activity after the fusion protein enters tumors, and thus IL-15 has great potential for development;
(4) the fusion protein can overcome the tolerance of PD-L1 antibody and has a synergistic effect when used in combination with PD-L1 antibody.

### BRIEF DESCRIPTION OF THE DRAWINGS

Figure 1 shows that the Anti-4-1BB-hIgG1 antibody could control the growth of small tumors, but was less effective in large tumors. 1A, inhibition effect of the Anti-4-1BB antibody on the growth of small tumors; 1B, inhibition effect of the Anti-4-1BB antibody on the growth of large tumors; 1C, depletion effect of the Anti-4-1BB antibody on the intratumoral Tregs; 1D, the flow cytometry plot of the depletion effect of the Anti-4-1BB antibody on the intratumoral Tregs.
Figure 2 shows anti-tumor effect and lymphocyte expansion effect of IL-15 at different doses. 2A, the high dose of IL-15 inhibited the tumor growth; 2B, IL-15 significantly expanded the peripheral and intratumoral immune cells; 2C, IL-15 treatment caused an increase in ALT level in the blood.
Figure 3 shows that the combination therapy of IL-15 and the Anti-4-1BB antibody had a synergistic effect. 3A, IL-15 could not overcome the inhibition of Tregs on CD8+T cells; 3B, the Anti-4-1BB antibody and IL-15 had synergistic anti-tumor effect.
Figure 4 shows construction and expression of Anti-4-1BB-MMP-IL-15-IL15Ra fusion protein. 4A, the structural pattern diagram of the Anti-4-1BB-MMP-IL-15-IL15Ra fusion protein; 4B, SDS-PAGE analysis of the constructed Anti-4-1BB-MMP-IL-15-IL15Ra fusion protein, wherein R represents a reducing band and NR represents a non-reducing band.

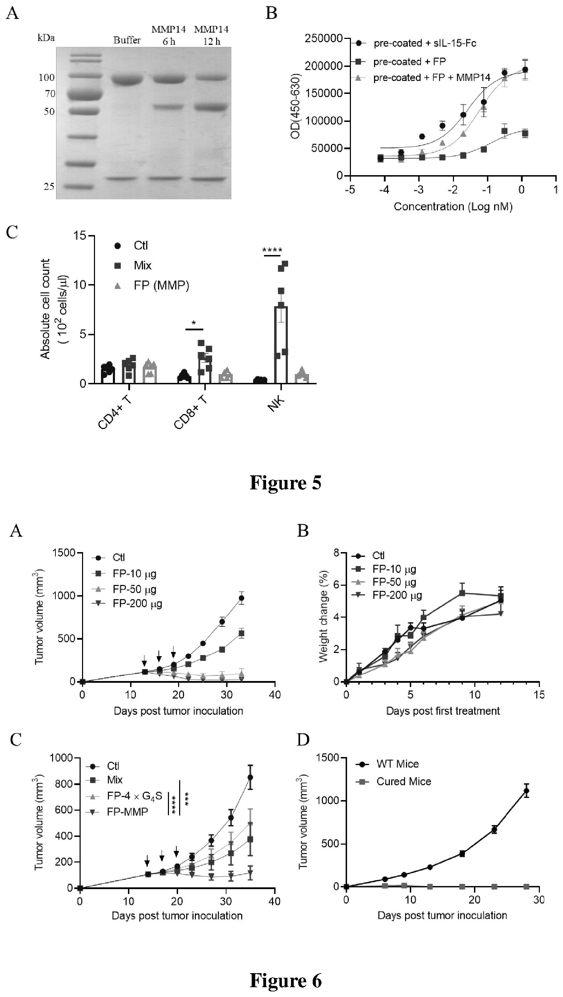

Figure 5 shows detection of *in vivo* and *in vitro* function of the Anti-4-1BB-MMP-IL-15-IL15Ra fusion protein. 5A, *in vitro* cleavage of the fusion protein by MMP, wherein Buffer represents the band of the fusion protein after incubation in the cleavage solution without MMP14 for 12 hours (h), and MMP14 6 h or MMP14 12 h band represents the protein band of the fusion protein after cleavage by MMP14 for 6 h or 12 h; 5B, activation of STATS signal pathway in CTLL-2 cell line by the fusion protein and the cleaved protein; 5C, the expansion effect of the fusion protein on immune cells in peripheral blood.
Figure 6 shows that the Anti-4-1BB-MMP-IL-15-IL15Ra fusion protein had a synergistic therapeutic effect. 6A, tumor inhibition effect of the Anti-4-1BB-MMP-IL-15-IL15Ra fusion protein at different doses (in MC38 tumor model); 6B, effect of the fusion protein at different doses on the weight of mice; 6C, comparison of therapeutic effect of the fusion protein, Mix and the fusion protein without MMP ecognizable substrate sequence (the MMP ecognizable substrate sequence was replaced with 2G4S); 6D, re-challenge results in the treatment group with Anti-4-1BB-MMP-IL-15-IL15Ra fusion protein (in MC38 tumor model).
Figure 7 shows that the therapeutic effect of Anti-4-1BB-MMP-IL-15-IL15Ra fusion protein was independent of innate immune cells, and the fusion protein had a therapeutic effect on Rag tumor-bearing mice.
Figure 8 shows that the function of Anti-4-1BB-MMP-IL-15-IL15Ra fusion protein depended on CD8 T cells. 8A, depletion efficiency of CD4 T cells and CD8 T cells in peripheral blood on Day 1 post injection of 200 µg of the depletion antibody, detected by flow cytometry; 8B, tumor growth curves of different treatment groups.
Figure 9 shows that intratumoral T cells played a key role in the treatment with Anti-4-1BB-MMP-IL-15-IL15Ra fusion protein, and the fusion protein had a therapeutic effect on tumor-bearing mice injected with FTY720.
Figure 10 shows that the immune response induced by local treatment of tumors *in situ* could control the growth of distal tumors. 10A, the therapeutic effect on tumors in the left and right (administration) sides in different treatment groups in the MC38 model; 10B, the therapeutic effect on tumor *in situ* in the B16F10 tumor model; 10C, the therapeutic effect on pulmonary metastatic tumor nodules in the B16F10 tumor model.
Figure 11 shows that Anti-4-1BB-MMP-IL-15-IL15Ra fusion protein therapy depleted intratumoral Tregs and enhanced the proportion of CD8 T cells to Tregs. 11A, flow cytometry plot of intratumoral Treg cells in different treatment groups; 11B, the proportion of intratumoral Tregs in CD4+ T cells in different treatment groups; 11C, flow cytometry plot of intratumoral CD8+T cells in different treatment groups; 11D, the proportion of intratumoral CD8+T in CD45+ cells in different treatment groups.
Figure 12 shows that the antibody function depended in part on the binding of Fc to FcyR.
Figure 13 shows that aPDL1 antibody could synergize with Anti-4-1BB-MMP-IL-15-IL15Ra fusion protein to improve the anti-tumor effect.
Figure 14 shows that the Anti-OX40-MMP-IL-15-IL15Ra fusion protein had a good anti-tumor effect.
Figure 15 shows detection of *in vitro* function of the humanized Anti-4-1BB-MMP-IL-15-IL15Ra fusion protein. 15A, SDS-PAGE detection of the humanized fusion protein; *in vitro* cleavage of the fusion protein by MMP, wherein Buffer represents the band of the fusion protein after incubation in a cleavage solution without MMP14 for a certain period of time, and MMP 6 h or 12 h band represents the protein band of the fusion protein after cleavage by MMP14 for 6 h or 12 h; 15B, activation of STATS signal in HEK-IL2 cell line by and EC50 of the fusion protein and the cleaved protein.
Figure 16 shows detection of activity of the humanized Anti-4-1BB-MMP-IL-15-IL15Ra fusion protein to expand immune cells in hu-PBMC mice.
Figure 17 shows therapeutic effect and safety of the humanized Anti-4-1BB-MMP-IL-15-IL15Ra fusion protein in hu-PBMC tumor-bearing mice. 17A, the anti-tumor effect of the fusion protein and mix treatment groups; 17B, weight change in the fusion protein and mix treatment groups.

### DETAILED DESCRIPTION

### Experimental materials

### 1. Strain and plasmid

Strain: Top10 E.coli and DH5α E.coli competent cells (Beijing TransGen Biotech Ltd.)

### Plasmid:

pEE12.4-IgGκ, containing a signal peptide of mouse IgGκ, for expression of cytokines and antibodies.

The primers used in the experiment were designed by DNAMAN software and synthesized by GENEWIZ Company.

### 2. Experimental animals

Wild type C57BL/6, BALB/c mice and BALB/c-nude mice were purchased from Beijing Vital River Laboratory Animal Center, China. Unless otherwise specified, all animals used in experiments were female mice aged 8-10 weeks.

The mice were fed in a specific pathogen-free (SPF) barrier environment. The feeding and experimental protocols of animals complied with the relevant regulations of Animal Care and Use Committee of Institute of Biophysics, Chinese Academy of Sciences.

### 3. Cell lines

MC38 is a colorectal cancer cell line derived from C57 mice.

CT26 is a colorectal cancer cell line derived from BALB/C mice.

The above cell lines were cultured in DMEM complete medium (containing 10% of inactivated fetal bovine serum, 2 mmol/l of L-glutamine, 0.1 mmol/l of non-essential amino acids, 100 U of penicillin and 100 µg/ml of streptomycin).

TIB-210TM hybridoma cell line (ATCCTIB-210), for expressing the depletion antibody for CD8+ T cells (clone: 2.43).

TIB-207TM hybridoma cell line (ATCC-TIB-207), for expressing the depletion antibody for CD4+ T cells (clone: GK1.5).

HB-197TM hybridoma cell line (ATCC-HB-197), for expressing an antibody blocking FcγRII/III in mice (clone: 2.4G2).

FreeStyleTM 293F cell line (Invitrogen), a suspension cell line, derived from HEK293 cell strain, cultured in SMM293-TII or CD OptiCHOTM medium, and mainly for transient transfection and expression of the fusion protein.

CTLL-2 cell line, a murine T cell line, for detecting the biological activity of IL-2.

The above cell lines were cultured in RPMI1640 complete medium (containing 10% of inactivated fetal bovine serum, 2 mmol/L of L-glutamine, 0.1 mmol/L of non-essential amino acids, 100 U of penicillin, 100 µg/ml of streptomycin and 100 IU/ml of recombinant IL-2).

### IL15&IL-15R Sushi

The mouse IL-15 has an amino acid sequence as shown in SEQ ID NO.7, and the mouse IL-15R Sushi domain has an amino acid sequence as shown in SEQ ID NO.8;
the human IL-15 has a amino acid sequence as shown in SEQ ID NO.13, and the human IL-15R Sushi region has a amino acid sequence as shown in SEQ ID NO.14;
sIL15 mentioned in the Examples corresponds to IL15-RA-Fc in CN201810420739.6, and has an amino acid sequence as shown in SEQ ID NO.15:

SEQ ID NO.15:
NWIDVRYDLEKIESLIQSIHIDTTLYTDSDFHPSCKVTAMNCFLLELQVILHEYSNMTLNE
TVRNVLYLANSTLSSNKNVAESGCKECEELEEKTFTEFLQSFIRIVQMFINTSSGGGSGGG
GSGGGGSGGGGSGGGSLQGTTCPPPVSIEHADIRVKNYSVNSRERYVCNSGFKRKAGTS
TLIECVINKNTNVAHWTTPSLKCIRDPSLAHYSPVPTGGGGSEPKSCDKTHTCPPCPAPEL
LGGPSVFLFPPKPKDQLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFLYSKLTV
DKSRWQQGNVFSCSVLHEALHNHYTQKSLSLSPGK.

### Fab region of Anti-4-1BB antibody

Light chain variable region + light chain constant region (light chain) of human or murine 4-1BB antibody;
heavy chain variable region + CH1 region (heavy chain) of human or murine 4-1BB antibody.

### Fc region of Anti-4-1BB antibody

Fc region (CH2+CH3) of human IgG1 or an Fc region variant of human IgG1 with ADCC effect knocked out.

### Mouse tumor inoculation and treatment

### (1) Tumor inoculation and measurement:

establishment of tumor model

5×10⁵ MC38 single cells were suspended in 100µL of PBS and inoculated subcutaneously into C57BL/6 mice on the back.

When the mice with regressed tumors were re-challenged with the same type of tumor cells, the number of the inoculated tumor cells was five times that in the initial tumor modeling, and the site of inoculation was subcutaneous on the other side of the back of the mice. The tumor size was monitored twice a week, by using a vernier caliper to measure the long diameter (a), short diameter (b) and height (c) of the tumor, and the mouse tumor volume = a×b×c/2.

### (2) Treatment:

The antibody or the antibody fusion protein was injected intraperitoneally, but also intratumorally in some experiments. The specific administration dosage will be described in the specific experiments.

### Preparation of monoclonal antibody (mouse ascites method)

The CD4⁺ T cell depletion-type antibody GK1.5, the CD8⁺ T cell depletion-type antibody TIB210 and the FcRII/III blockade antibody used in the experiments were all derived from corresponding hybridoma cells (TIB-210TM, TIB-207TM and HB-197TM), and were produced and purified in our laboratory.

### Cell depletion in mice

### Depletion of CD4⁺ T cells and CD8⁺ T cells:

The day before the treatment with the fusion protein, 200 µg of GK1.5 or TIB210 antibody were injected intraperitoneally to deplete CD4⁺ T cells or CD8⁺ T cells, and then injected every 4 days. The number of injections was adjusted based on the treatment period. The depletion efficiency was determined by flow cytometry.

### Blockade of T cell migration

FTY720 (purchased from Sigma Company) is an immunosuppressant that can reduce the migration of T cells from lymphoid organs to peripheral blood circulation.

In the present disclosure, FTY720 blockade was performed at different periods of mouse tumor inoculation to change the tumor microenvironment. Blockade was performed during the treatment of tumor in mice: 20 µg of FTY720 was injected intraperitoneally one day before tumor treatment, and then 10 µg was injected intraperitoneally every other day. The blocking time was determined based on the treatment period, which resulted in no T cells newly migrating into the tumor tissue during tumor treatment. With the FTY720 blockade regimen, the importance of infiltrating lymphocytes in tumor tissue could be studied.

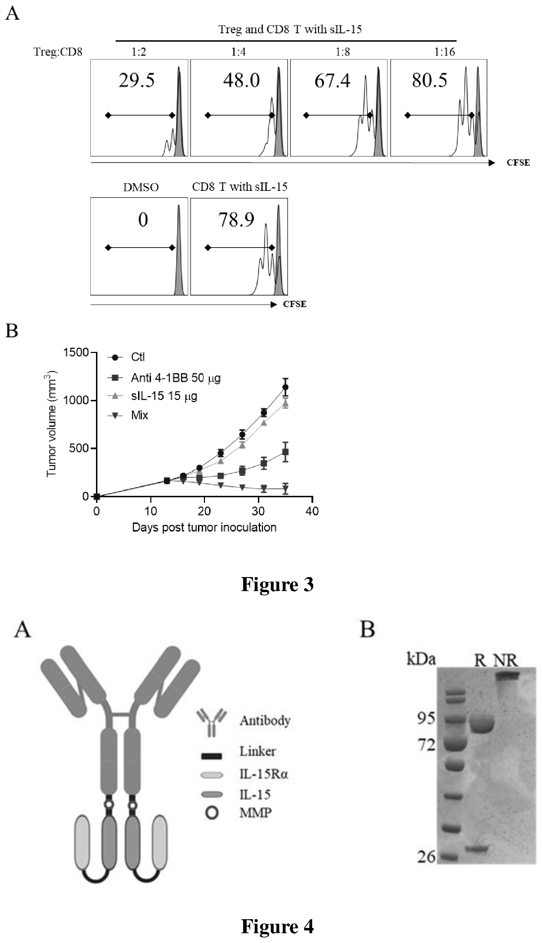

**Unless otherwise specified, the molecular structure of Anti-4-1BB-MMP-IL-15-IL15Ra fusion protein in the following Examples 1-5 is shown in** **Figure 4A****.**

### Example 1. Significantly improved effect of Anti-4-1BB-MMP-IL-15-IL15Ra fusion protein compared with separate treatments

### 1. Anti-4-1BB antibody effective in small tumors but not in large tumors

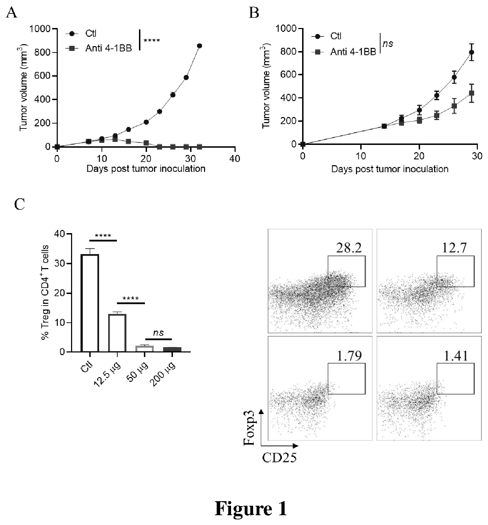

A human IgG1 isotype Anti-4-1BB antibody was used to verify the antibody function in the MC38 tumor model. Compared with the untreated control group, the antibody treatment group could effectively control the growth of small tumors (**Figure 1A**), but failed to completely eliminate the tumors when the tumors were large (**Figure 1B**). Different doses of the 4-1BB antibody could eliminate the intratumoral Tregs to a different extent (**Figure 1C**).

C57BL/6 mice were inoculated with 5×10⁵ MC38 tumor cells subcutaneously, and the tumor-bearing mice were treated in different groups (n=5/group) on Day 7 or 12 by intraperitoneally injecting (ip) 200 µg of the Anti-4-1BB antibody every three days for a total of three times.

The results showed that the treatment by administering the antibody alone had a good anti-tumor effect only on small tumors.

### 2. Anti-tumor effect and lymphocyte expansion effect of IL-15 at different doses

Given the fact that the human IgG1 isotype 4-1BB antibody can effectively deplete the intratumoral Tregs cells, which are a type of intratumoral immunosuppressive cells that have a great inhibitory effect on the function of T cells, while the Anti-4-1BB antibody cannot treat advanced tumors, the function of T cells needs to be further exerted. The depletion of Tregs relieved the inhibition of T cells, but the number of T cells did not change significantly. To effectively expand CD8⁺T cells, IL-15 was used as a helper to expand T cells. To verify the expansion effect of IL-15, 10 µg and 50 µg of sIL-15, i.e., the fusion protein in the IL-15-IL15Ra-Fc form, was used to treat MC38 tumor-bearing mice every 3 days for 3 consecutive times. Spleens and tumor tissues of mice were collected on Day 2 post the second injection to detect the number and proportion of immune cells. ALT levels in peripheral blood of mice were detected on Day 7 post the last injection.

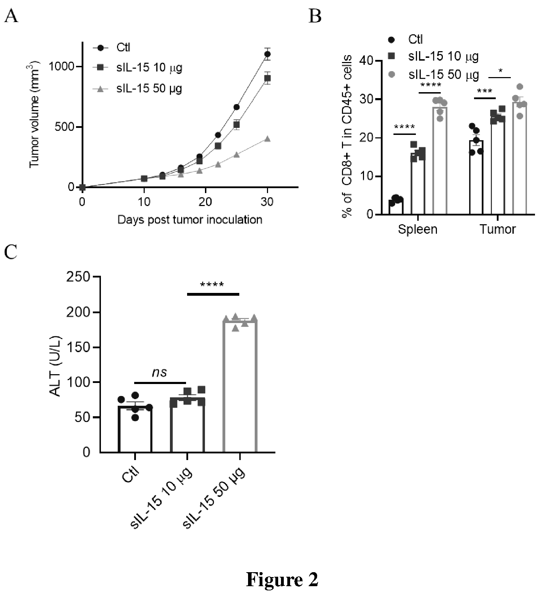

The results showed that the mice in the 50 µg-dose treatment group had a significant difference in tumor volume compared with the untreated group, while the mice in the 10 µg-dose treatment group had no significant difference in tumor volume compared with the untreated group (Figure 2A). Both the low and high dose groups significantly increased the proliferation of CD8⁺T cells in the spleens and tumors (**Figure 2B**)**.** ALT levels in peripheral blood of mice were significantly increased post IL-15 treatment (**Figure 2C**).

### 3. Synergistic effect of the combination therapy of IL-15 and Anti-4-1BB

IL-15 has a good expansion effect on T cells, but Tregs cells have a strong inhibitory effect on the proliferation of T cells. To detect whether the expansion of IL-15 on T cells can overcome the inhibitory effect of Tregs *in vitro,* the anti-CD3 antibody was used to stimulate CFSE-labeled CD8⁺T cells *in vitro,* 1 µg/mL of sIL-15 was added, and different proportions of Tregs were added simultaneously. The CFSE intensity change in CD8⁺T cells and the proliferation proportion of T cells were detected post 3 days of incubation.

The results showed that the addition of IL-15 could not overcome the inhibitory effect of Tregs on the proliferation of CD8⁺T cells. Compared with the groups with no Tregs added, the proliferation proportion of T cells was reduced post the addition of Tregs (**Figure 3A**) (the light-colored peaks in the figure represents the number of proliferation generations of CD8⁺T cells, which was significantly reduced as the number of the added Treg cells increased).

Given that the 4-1BB antibody and IL-15 *per se* have a limited therapeutic effect, but are complementary to each other in function, we combined both to test whether they are synergistic in anti-tumor effect. MC38 tumor-bearing mice were given the combination therapy from Day 13 post tumor inoculation, by injecting intraperitoneally 200 µg of the Anti-4-1BB antibody and 15 µg of sIL-15 every 3 days for 3 consecutive times, and then measured for the tumor volume.

The results showed that the combination of the Anti-4-1BB antibody and sIL-15 had a superior effect to that of their respective monotherapy and had a synergistic anti-tumor effect (**Figure 3B**).

### 4. Synergistic anti-tumor effect of Anti-4-1BB-MMP-IL-15-IL15Ra fusion protein

### 4.1. Construction and expression of the fusion protein

Although the combination therapy of the Anti-4-1BB antibody and sIL-15 has a synergistic anti-tumor effect, sIL-15 treatment causes peripheral toxicity. Taking this into consideration, it is aimed to design a fusion protein that can both act as an antibody to deplete the intratumoral Tregs and expand the intratumoral T cells with sIL-15, and make IL-15 exert activity within tumors as much as possible, without expanding immune cells in the periphery so as to avoid peripheral toxicity.

We designed a fusion protein by linking IL-15-IL-15Ra to the antibody at the carboxyl terminal by a short linker, which blocks the activity of IL-15. When the short linker is replaced with a substrate sequence that can be recognized by highly expressed MMP in tumors, the fusion protein can be cleaved within tumors to release IL-15 activity. Each molecule comprises a complete heavy chain of the Anti-4-1BB antibody and the IL-15-IL-15Ra molecule, with an MMP substrate sequence there between (**Figure 4A**). 293F cells were co-transfected with the antibody light chain plasmid and the fusion protein plasmid. Seven days later, the cell supernatant was collected, centrifuged and subjected to protein purification with protein A column, and the purified antibody was identified by protein gel. The protein was analyzed for molecular composition by SDS-PAGE, which proved that the fusion protein could express the light chain and the fused heavy chain of the Anti-4-1BB antibody (**Figure 4B**).

### 4.2. Function verification of the fusion protein

### (1) Detection of in vitro activity of the fusion protein

As the MMP substrate sequence in the fusion protein can be cleaved by MMP2, MMP9 or MMP14, the fusion protein and the activated MMP14 enzyme were co-incubated *in vitro,* and then subjected to SDS-PAGE analysis to verify *in vitro* cleavage of the fusion protein. The results showed that the fusion protein could be effectively cleaved *in vitro* (**Figure 5A**). IL-15, upon binding to its receptor, often induces the activation of the downstream STATS signal pathway. The CTLL2-STAT5-Luc cell line was used. The 4-1BB molecules expressed in the CTLL-2 cells were blocked by the Anti-4-1BB antibody of the same clonal strain, and then the cells were stimulated by adding different concentrations of the fusion protein. After 4 hours, the cells were detected for luciferase activity. The results showed that the ability of the fusion protein to activate the STATS signal pathway was decreased, but was completely restored after the fusion protein was enzymatically cleaved *in vitro* (**Figure 5B**), indicating that the activity of IL-15 in the fusion protein is well blocked, and is fully restored after MMP cleavage.

### (2) Detection of the expansion activity of the fusion protein on immune cells in mice

The activity of IL-15 in the fusion protein is well blocked. To further verify whether the fusion protein expand immune cells in peripheral tissues of tumor-bearing mice, 100 µg of the fusion protein or an equimolar amount of the Anti-4-1BB antibody and sIL-15 mix was injected intraperitoneally every three days. The numbers of CD4⁺T cells, CD8⁺T cells and NK cells in peripheral blood were measured on Day 2 post the second treatment. The results showed that, compared with the mix treatment group, the fusion protein did not cause substantial expansion of T cells and NK cells in peripheral blood and had good safety (**Figure 5C**).

### 4.3. Good safety and anti-tumor effect of the fusion protein

C57BL6 mice were inoculated with 5×10⁵ MC38 tumor cells subcutaneously, and were given treatment from D13 post tumor inoculation, by intraperitoneally injecting 10, 50 and 200 µg of the fusion protein, respectively. The treatments were performed on D13, D16 and D19, and the tumor size was measured twice a week.

The results showed that the therapeutic effect of the fusion protein was gradually improved with the increase of therapeutic dose of the fusion protein (**Figure 6A**).

Meanwhile, the weight of the mice was measured every 1-2 days before and after treatment to verify the effect of the fusion protein on the weight of the mice.

The results showed that the weight of the mice did not change significantly with the increase of the used dose (**Figure 6B**).

In addition, we compared the anti-tumor effect of 50 µg of the fusion protein, an equimolar amount of the Anti-4-1BB antibody and sIL-15 mix, and the fusion protein in which the MMP sequence was replaced with a 2×G₄S linker. The results showed that the fusion protein had a therapeutic effect superior to that of the mix therapy and the fusion protein without the MMP-recognizable substrate sequence sequence (**Figure 6C**).

Further, in the treatment group with the fusion protein, the tumor re-challenge experiment was performed two months post tumor elimination. In the treatment group with the fusion protein, the tumor re-challenge experiment was performed two months post tumor regression in the mice in which the tumors were completely eliminated. Three times the dose (1.5×10⁶) of MC38 tumor cells were inoculated subcutaneously, and only the tumor cells were inoculated in the re-challenge experiment. After inoculation, all groups were no longer given treatment.

The results showed that, compared to the control group, re-inoculation of three times the dose of MC38 cells did not cause tumor growth, indicating that the treatment with the fusion protein induces the production of memory immune cells in mice (**Figure 6D**).

### Example 2. Activation of CD8 T cells by Anti-4-1BB-MMP-IL-15-IL15Ra fusion protein

### 1. The therapeutic effect of Anti-4-1BB-MMP-IL-15-IL15Ra fusion protein independent of innate immune cells

To explore which population of cells mediates the anti-tumor function of the fusion protein, mice with Rag1 gene knocked out were used. Due to the lack of Rag gene, TCR and BCR cannot rearrange in such mice, and thus the T and B cells cannot develop into mature stage, resulting in lack of T and B lymphocytes.

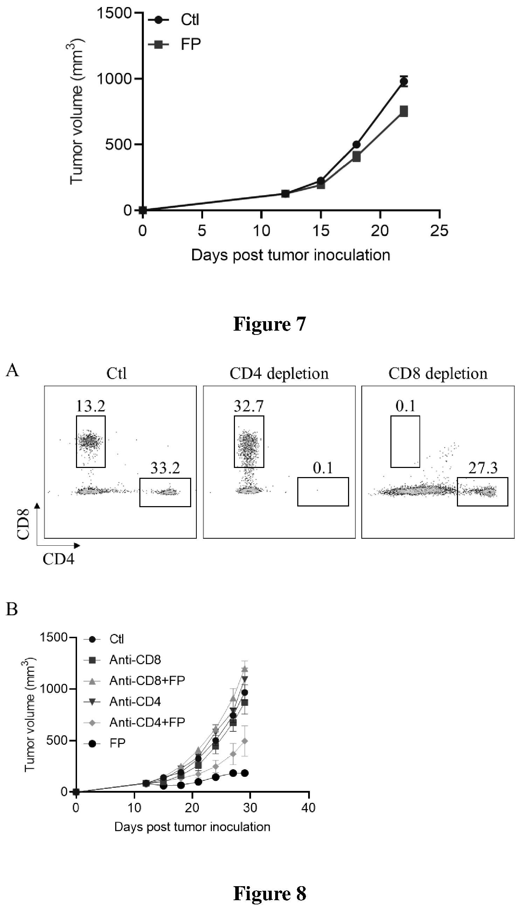

Rag1-/- mice were inoculated with 5×10⁵ MC38 tumor cells subcutaneously, and 50 µg of the fusion protein was injected intraperitoneally on Days 12, 15 and 18 to verify whether the therapeutic effect of the fusion protein depends on innate immune cells or adaptive immune cells. The fusion protein was found to have no anti-tumor effect in Rag1-/- mice which had received the treatment (**Figure 7**), indicating that the effect of the fusion protein depends on adaptive immune cells.

### 2. The therapeutic effect of Anti-4-1BB-MMP-IL-15-IL15Ra fusion protein dependent on CD8⁺T cells

We further verified the role of T cells in the treatment with the fusion protein.

The treatment was initiated on Day 12 post subcutaneous inoculation of 5×10⁵ MC38 tumor cells into C57BL/6 mice on the back, by intraperitoneally injecting 50 µg of Anti-4-1BB-MMP-IL-15-IL15Ra (administration on Days 12, 15 and 18 post tumor inoculation). One day before the treatment, 200 µg of a CD4T cell-depleting antibody (Clone No. GK1.5, prepared in our laboratory) and 200 µg of a CD8T cell-depleting antibody (Clone No. TIB210, prepared in our laboratory) were injected intraperitoneally for a total of four times.

CD8⁺T cells and CD4⁺T cells were depleted by the anti-CD8and anti-CD4 monoclonal antibodies, respectively, and the depletion efficiency was detected. The results showed that the antibodies were effective in depleting T cells in peripheral blood (**Figure 8A**). Depletion of CD4⁺T cells during the treatment with the fusion protein did not affect the anti-tumor effect of the fusion protein, while the anti-tumor effect disappeared completely post depletion of CD8⁺T cells (**Figure 8B**).

### 3. The therapeutic effect of Anti-4-1BB-MMP-IL-15-IL15Ra fusion protein dependent on the intratumoral CD8⁺T cells

To further explore whether the fusion protein-dependent CD8+T cells that perform their roles are the population of T cells existing in tumors *per se,* or the T cells outside tumors which are activated by the fusion protein and then migrate into tumors, FTY720, an inhibitor that inhibits T cell migration from lymph nodes to the periphery, was used to block the T cell migration from lymph nodes to tumors, thereby maintaining the stability of intratumoral T cells.

### The experimental protocols were as follows:

C57BL/6 mice were inoculated with 5×10⁵ MC38 tumor cells subcutaneously on the back, and then were intraperitoneally injected with 50 µg of the fusion protein on Days 12, 15 and 18 post tumor inoculation. In the treatment group with FTY720, FTY720 was injected intraperitoneally at 15 µg on Day 11 post tumor inoculation and then was injected at 10 µg on Days 13, 15, 17, 19 and 21, respectively.

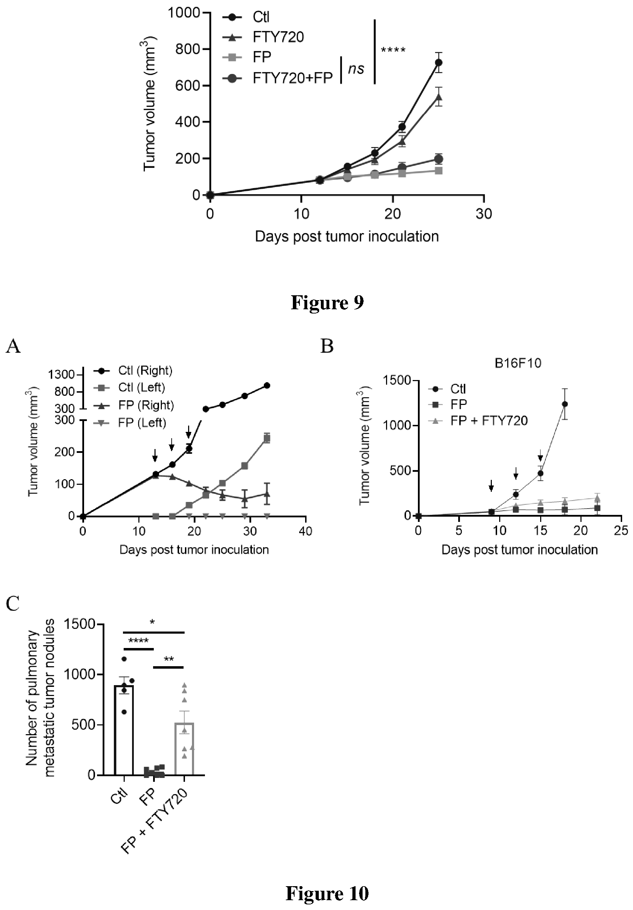

The treatment with the fusion protein remained effective post FTY720 blockade (**Figure 9**). The above results indicates that the anti-tumor effect of the fusion protein depends on CD8⁺T cells already present in tumors.

### 4. Protection effect of the treatment with Anti-4-1BB-MMP-IL-15-IL15Ra fusion protein on distal metastatic tumors

To verify whether the immunoprotection produced from the treatment of tumors *in situ* also has an inhibitory effect on tumors at other metastatic sites, we conducted an experiment in a double-tumor mouse model. The mice were inoculated subcutaneously with tumor cells on both the left and right sides and only one side was treated *in situ* to observe whether the distal tumor can also be controlled.

### The experimental protocols were as follows:

C57BL6 mice were inoculated with 5×10⁵ MC38 tumor cells on the right side of the back and received intratumoral local treatment with 30 µg of the fusion protein on Days 13, 16 and 19 post tumor inoculation. On Day 13, the mice were inoculated with 1×10⁶ MC38 tumor cells on the left side. The tumor volume on both sides was measured twice a week.

The results showed that the treatment *in situ* of the right tumor only by intratumoral injection could not only eliminate the tumor *in situ,* but also control the untreated distal tumor on the opposite side (**Figure 10A**).

In addition, a B16F10 mouse tumor model was used to further verify the therapeutic effect of the treatment with the fusion protein on pulmonary metastatic tumor nodules during melanoma metastasis.

### The experimental protocols were as follows:

C57BL6 mice were inoculated with 5×10⁵ B16F10 tumor cells on the right side of the back and received intratumoral local treatment with 30 µg of the fusion protein on Days 9, 12 and 15 post tumor inoculation, and 15 µg of FTY720 was injected intraperitoneally every 2 days from Day 8 post tumor inoculation until the end of experiment. On Day 8 post tumor inoculation, 5×10⁵ B16F10 tumor cells were injected into the tail vein of each mouse intravenously. On Day 10 post the third treatment, the mice were perfused, the lungs of the mice were taken, and the melanoma metastatic tumor nodules in the lung were counted to verify the therapeutic effect of the treatment with the fusion protein on the metastatic tumor nodules.

The results showed that the intratumoral treatment with the fusion protein had a significant anti-tumor effect on the subcutaneous tumors *in situ* and could partially eliminate the tumors *in situ,* and the blockade of the T cell migration by FTY720 did not affect the control of the tumors *in situ* (**Figure 10B**). After FTY720 blockade, the pulmonary metastatic tumor nodules in the treatment group with the fusion protein were similar to those in the untreated control group, while the number of the pulmonary metastatic tumor nodules in the treatment group with the fusion protein was significantly reduced (**Figure 10C**).

### Example 3. Depletion of intratumoral Tregs and increase of the proportion of CD8/Treg cells by Anti-4-1BB-MMP-IL-15-IL15Ra fusion protein

### 1. Depletion of intratumoral Tregs and increase of the proportion of CD8/Treg cells by Anti-4-1BB-MMP-IL-15-IL15Ra fusion protein

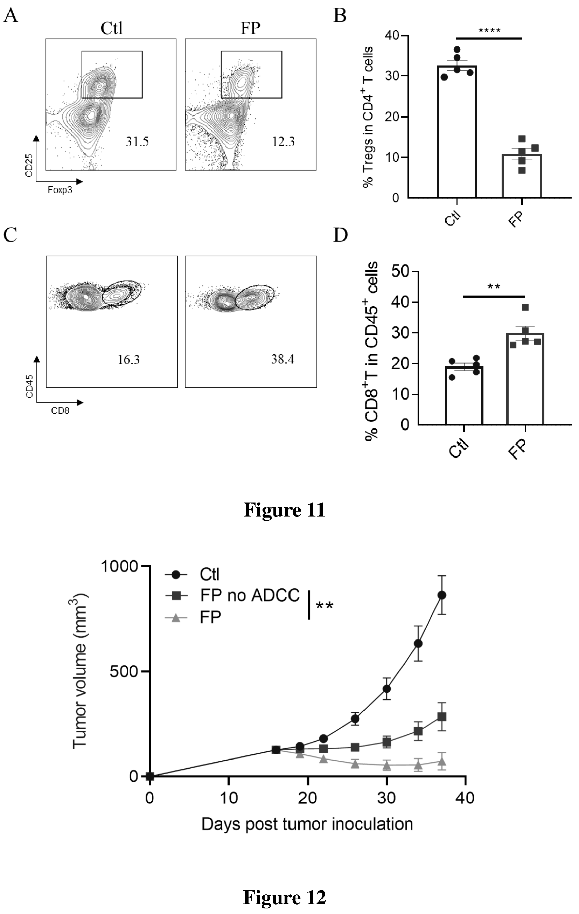

The human IgG1 isotype Anti-4-1BB antibody can effectively deplete intratumoral Tregs, and IL-15 can expand CD8+T cells. To verify the function of the fusion protein *in vivo,* MC38 tumor-bearing mice were treated on Days 13 and 16 post tumor inoculation by intraperitoneally injecting 50 µg of Anti-4-1BB-MMP-IL-15-IL15Ra fusion protein. The tumor tissues were taken on Day 18, and subjected to flow cytometry for the proportion of intratumoral Tregs and CD8+T cells. By the flow cytometry analysis, we found that Anti-4-1BB-MMP-IL-15-IL15Ra fusion protein could effectively deplete intratumoral Treg cells and increase the proportion of CD8 T cells, thus having the best therapeutic effect (**Figure 11A-D**).

### 2. The therapeutic effect of Anti-4-1BB-MMP-IL-15-IL15Ra fusion protein dependent on the binding of Fc to FCγR

The analysis of intratumoral lymphocytes in MC38 tumor-bearing mice showed that Anti-4-1BB-MMP-IL-15-IL15Ra fusion protein has the function of depleting Tregs. To verify whether Treg depletion is the main mechanism by which Anti-4-1BB-MMP-IL-15-IL15Ra fusion protein exerts anti-tumor effect, we constructed Anti-4-1BB-MMP-IL-15-IL15Ra-no ADCC antibody by site mutation in the Fc region. The mutated Fc does not bind to FCγR receptor and thus loses the ADCC or ADCP-mediated depletion function, but still retains the expansion effect of IL-15 on immune cells. A MC38 tumor model was used. C57BL6 mice were inoculated with MC38 tumor cells and received the treatment on Day 13 post inoculation. 50 µg of antibodies with wt Fc or with no ADCC Fc was injected intraperitoneally every three days for a total of three times.

The results showed that, after tumor inoculation, the therapeutic effect of the antibody with wt Fc was compared with that of the antibody with no ADCC Fc, and it was found that the mutated Fc reduced the therapeutic effect of the fusion protein (**Figure 12**). In the treatment group with Anti-4-1BB-MMP-IL-15-IL15Ra-no ADCC, the fusion protein still retained some anti-tumor effect, indicating that Anti-4-1BB-MMP-IL-15-IL15Ra fusion protein performs its function mainly depending on the depletion of Treg cells.

### Example 4. The therapy of Anti-4-1BB-MMP-IL-15-IL15Ra combined with PDL1 antibody

### 1. Up-regulation of PDL1 in intratumoral immune cells post the treatment with the fusion protein

When MC38 tumor-bearing mice are treated, the tumor can be effectively controlled, but cannot be eliminated in some mice. Where the tumor is relatively large, the treatment with the fusion protein alone can only control the tumor growth. The treatment with the fusion protein often activates intratumoral immune cells and the improvement of activation degree often results in a negative feedback of the immune system, causing up-regulated expression of immunosuppressive checkpoint molecules, and thus inhibiting the activation of immune cells. To better improve the therapeutic effect, the mice, upon treatment with the fusion protein, were detected for the expression of PD-1 and PD-L1 in intratumoral immune cells, for the purpose of finding an appropriate immune checkpoint blockage antibody, which is used as a combined therapy to observe whether the therapeutic effect can be improved.

Protocols: C57BL/6 mice were inoculated with 5×10⁵ MC38 tumor cells subcutaneously on the back, and received the treatment with 50 µg of the fusion protein on Days 12 and 15. Tumor tissues of the mice were taken on Day 18 to analyze the expression of PD-1 and PD-L1 in intratumoral immune cells.

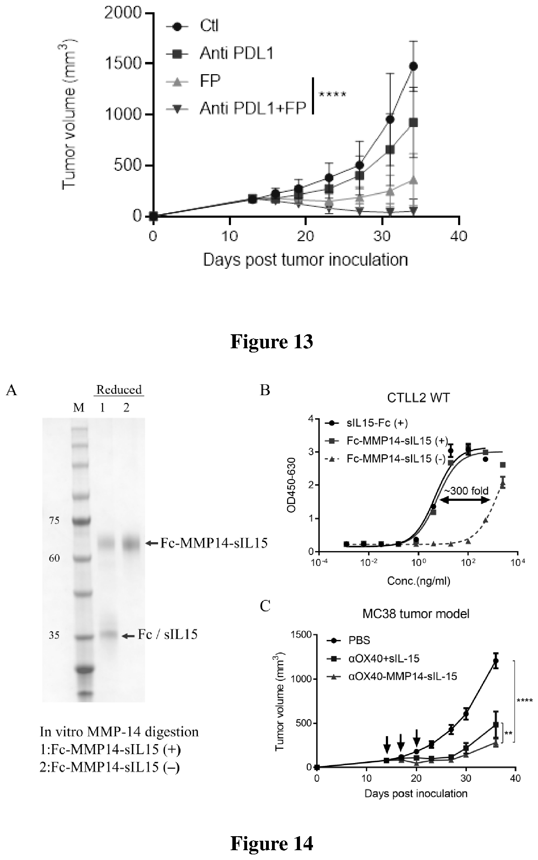

The results showed that, the mice, upon treatment, had significantly increased PD-L1 expression in intratumoral CD45⁺ immune cells, but significantly decreased PD-1 expression in CD8⁺T cells (**Figure 13**), which suggests that the fusion protein and the PD-L1 antibody can be used in combination to further relieve the degree of intratumoral immunosuppression. To further improve the therapeutic efficacy of the fusion protein, the fusion protein and an PD-L1 antibody were used to treat advanced tumor-bearing mice (with tumor volume of about 150-200 mm³).

Protocols: C57BL/6 mice were inoculated with 1×10⁶ MC38 tumor cells subcutaneously on the back, 200 µg of the aPDL1 antibody was injected intraperitoneally on Days 13 and 16, and 50 µg of Anti-4-1BB-MMP-IL-15-IL15Ra fusion protein was injected intraperitoneally on Days 13, 16 and 19.

The results showed that, for advanced tumors, the PD-L1 antibody could further improve the therapeutic effect of the fusion protein and overcome immune tolerance.

Finally, it should be noted that the above examples are intended only to assist those skilled in the art in understanding the essence of the present disclosure and not intended to limit the scope of the present disclosure.

### Example 5. Good anti-tumor effect of Anti-OX40-MMP-IL-15-IL15Ra fusion protein

### 1. Construction and expression of the fusion protein

The above studies had proved that the combination therapy of Anti-4-1BB antibody and sIL-15 has a synergistic anti-tumor effect. Given that both OX40 and 4-1BB are members of TNF superfamily and also a T cell activating factor, it was speculated that the Anti-OX40 antibody, upon fusion with sIL-15, might also produce synergistic anti-tumor effect. Here, we once again designed a fusion protein by linking IL-15-IL-15Ra to the immunoglobulin Fc at the carboxyl terminal through a short substrate sequence which can be recognized by MMP highly expressed in tumors between the Fc and IL-15, so that the fusion protein can be cleaved in tumors to release IL-15 activity. Each molecule comprises a complete Fc of the antibody, an IL-15-IL-15Ra molecule, and an MMP substrate sequence between the antibody and the cytokine. The fusion protein and the activated MMP14 enzyme were co-incubated *in vitro,* and then subjected to SDS-PAGE analysis to verify the *in vitro* cleavage of the fusion protein. The results showed that the fusion protein could be effectively cleaved *in vitro* (**Figure 14A**).

### 2. Detection of in vitro activity of the fusion protein (lymphocyte proliferation experiment (CCK8 assay))

CTLL2 cells were cultured in 1640 complete medium containing 100 U/ml of commercial recombinant IL2 cytokine for 24 hours, washed with the complete medium without IL2 for 2~3 times, and then were diluted to 2×10⁴/ml; samples of sIL15-Fc, Fc-MMP14-sIL15 (not cleaved by MMP14) and Fc-MMP14-sIL15 (cleaved by MMP14) were diluted in the complete culture medium without IL2 at a 5-fold dilution from the initial concentration of 5 µg/ml, with 10 dilution gradients; 100 µl of the cell suspension and 100 µl of the sample were added into a 96-well cell culture plate, and pipetted homogenously by a pipette tip; the cells were cultured for 72 hours, added with 20 µl of CCK8, and then cultured for further 3 hours; OD values at 450 nM and 630 nM were measured by a microplate reader.

The results showed that: (1) Fc-MMP14-sIL-15(-) had a biological activity which was about 300 times different from that of sIL15Fc, indicating that Fc-MMP14-sIL15 had a very low biological activity when it was not cleaved by MMP14; (2) Fc-MMP14-sIL-15(+) had a biological activity which was comparable to that of sIL15Fc, indicating that Fc-MMP14-sIL15 had a biological activity which was restored to the level of sIL15-Fc when it was cleaved by MMP14 (**Figure 14B**).

### 3. Good antitumor effect of the fusion protein

C57BL/6 mice were inoculated with 5×10⁵ MC38 tumor cells subcutaneously until the tumor grew to 100 mm³ and treated with 15 µg of αOX40, 15 µg of sIL15-Fc or 30 µg of αOX40-MMP14-sIL15 every three days for 3 times, and PBS was given for the control group in the same way. The tumor volume was measured (volume = length × width × height / 2).

The results showed that the tumor was well controlled by intraperitoneal administration of αOX40-MMP14-sIL15. This indicates that the fusion protein has a better anti-tumor effect than an equimolar mass of αOX40 and sIL15-Fc administered by combination (**Figure 14C**).

### Example 6. Good anti-tumor effect and safety of the humanized Anti-4-1BB-MMP-IL-15-IL15Ra fusion protein

### 6.1 Construction and expression of the humanized fusion protein

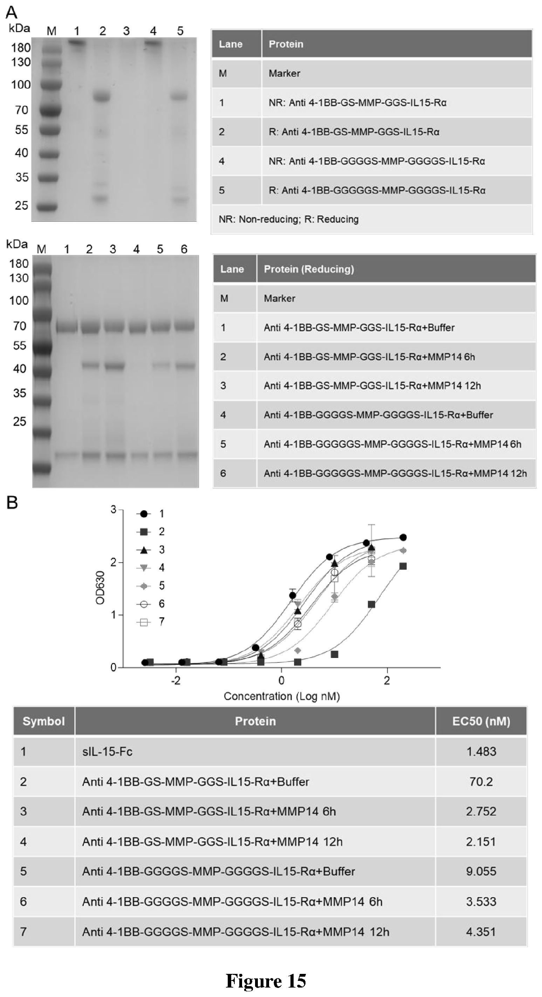

To verify that humanized Anti-4-1BB-MMP-IL-15-IL15R fusion protein had a similar anti-tumor effect, the fusion protein was constructed with the heavy chain of the antibody capable of recognizing human 4-1BB molecule and human IL-15-IL-15Ra in the same way. Two fusion proteins comprising linkers of different lengths, G₄S-MMP-G₄S and GS-MMP-GGS, were constructed, and then subjected to SDS-PAGE analysis (**Figure 15A**) (R: Reducing gel, NR: Non-reducing gel).

### 6.2. Function verification of the fusion protein

### (1) Detection of in vitro activity of the fusion protein

The MMP substrate sequence in the humanized fusion protein can be cleaved by MMP2, MMP9 or MMP14. The fusion protein and the activated MMP14 enzyme were co-incubated *in vitro,* and then subjected to SDS-PAGE analysis to verify *in vitro* cleavage of the fusion protein. The results showed that the fusion proteins with linkers of different length could be effectively cleaved *in vitro* (**Figure 15A**). HEK-IL2 cell line was used to detect activation of STATS signal in this cell line by human IL-15 comprised in the fusion protein. The results showed that the uncleaved fusion protein had a decreased ability to activate downstream signal in HEK-IL2 cell line. Upon enzymatically cleavage *in vitro,* the activation ability was substantially restored (**Figure 15B**), indicating that the activity of IL-15 in humanized fusion protein is well blocked, and its activity is fully restored post MMP cleavage.

### (2) The expansion of immune cells in hu-PBMC mice by the fusion protein

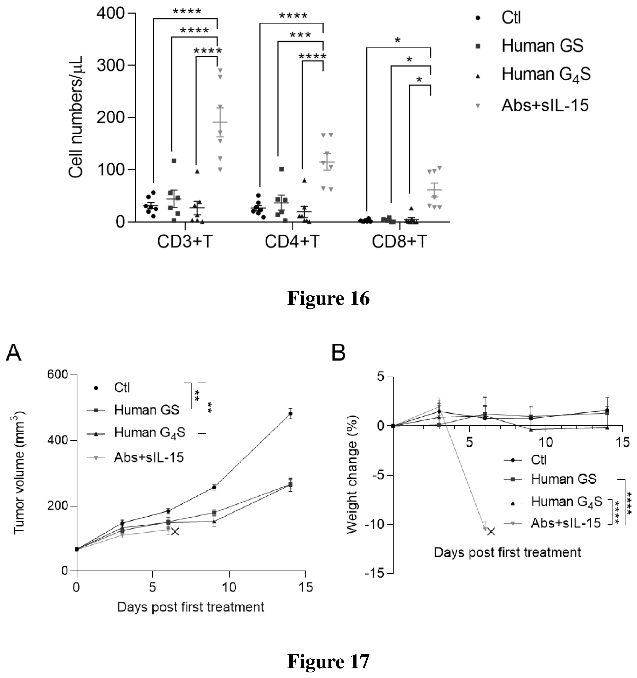

The activity of IL-15 in humanized fusion protein is well blocked. To further verify whether the fusion protein will expand human immune cells in peripheral tissues of tumor-bearing PBMC mice, 100 µg of the fusion protein or an equimolar amount of Anti-4-1BB antibody and sIL-15 mix was injected intraperitoneally every three days, and the numbers of CD4⁺T cells, CD8⁺T cells and CD3⁺T cells in peripheral blood were measured on Day 2 post the second treatment. The fusion protein with GS-MMP-GGS as linker is represented as Human GS, the fusion protein with G₄S-MMP-G₄S as linker is represented as Human G₄S, and the mix treatment group is represented as Abs+sIL-15. The results showed that, compared with the mix treatment group, the fusion protein did not substantially expand T cells in peripheral blood and had good safety (**Figure 16**).

### 6.3. Good safety and anti-tumor effect of the fusion protein

NSG mice are immunodeficient mice obtained by knockout mutations of Prkdc gene and Il2rg gene, which lack mature T, B and NK cells, do not produce immunoglobulin, and have dendritic cells (DCs) having abnormal function, and are very suitable for transplantation of human cells or tissues. NSG mice aged 6-8 weeks were inoculated with 1×10⁶ CFPAC1 cells subcutaneously on the right side of the back on D0 and were inoculated with 5×10⁶ PBMC cells by tail vein injection after 7 days. The mice were grouped based on the tumor size on Day 7 post inoculation of PBMC cells, and the tumor-bearing hu-PBMC mouse model was established.

100 µg of the fusion protein and an equimolar amount of Anti-4-1BB antibody and sIL-15 mix were injected intraperitoneally, respectively. The mice were treated on D14, D17 and D20 for three times, and measured for tumor size and weight twice a week. The results showed that, the fusion protein had a therapeutic effect superior to that of the control group (**Figure 17A**) and did not cause weight loss, while the mice in the mix treatment group post the second treatment lost weight of more than 10% and died, indicating that the safety of the fusion protein is superior to that of the mix treatment group (**Figure 17B**). The × in Figures 17A and B indicates that all the mice in the mix treatment group died.

## Claims

1. A bifunctional fusion protein composed of IL-15 and an antibody against a T cell co-stimulatory molecule, wherein the fusion protein comprises:
(1) an antibody against a T cell co-stimulatory molecule;
(2) a conjugate of IL-15 and IL-15R Sushi domain linked by a second linker; and
(3) a matrix metalloproteinase-cleavable first linker for linking the Fc region of the heavy chain of the antibody against the T cell co-stimulatory molecule to the conjugate of IL-15 and IL-15R Sushi domain;
the T cell co-stimulatory molecule is 4-1BB, ICOS or OX40, preferably 4-1BB; preferably, the antibody against the T cell co-stimulatory molecule is an IgG1-type antibody;
preferably, the first linker comprises 2-4 GnS linking units and a matrix metalloproteinase-recognizable and -cleavable short peptide contained therein, wherein n is an integer of 1-4.

2. The bifunctional fusion protein of claim 1, wherein
the matrix metalloproteinase-recognizable and -cleavable short peptide has an amino acid sequence as shown in SEQ ID NO.6; and
the first linker has an amino acid sequence as shown in SEQ ID NO.12.

3. The bifunctional fusion protein of claim 1, wherein the second linker has 2-5 G₄S linking units; preferably, the second linker has an amino acid sequence as shown in SEQ ID NO.5.

4. The bifunctional fusion protein of any one of claims 1-4, wherein the bifunctional fusion protein comprises:
(1) a first structural unit composed of a heavy chain of a 4-1BB antibody, the first linker and the conjugate of IL-15 and IL-15R Sushi domain from N-terminus to C-terminus;
(2) a second structural unit composed of a light chain of the 4-1BB antibody paired with the heavy chain of the 4-1BB antibody;
and the bifunctional fusion protein is dimerized via the Fc region of the heavy chain of the 4-1BB antibody to form a homologous dimer.

5. The bifunctional fusion protein of claim 4, wherein
the 4-1BB antibody is an antibody formed by fusing the Fab region of a human or murine antibody with the Fc region of IgG1,
the heavy chain (VH+CH1) of the murine 4-1BB antibody has an amino acid sequence as shown in SEQ ID NO.2;
the heavy chain (VH+CH1) of the human 4-1BB antibody has an amino acid sequence as shown in SEQ ID NO.10;
the light chain (VL+CL) of the murine 4-1BB antibody has an amino acid sequence as shown in SEQ ID NO.1;
the light chain (VL+CL) of the human 4-1BB antibody has an amino acid sequence as shown in SEQ ID NO.9; and
the Fc region of IgG1 is the Fc region of human IgG1 or a Fc region variant of human IgG1 with ADCC effect knocked out.

6. The bifunctional fusion protein of claim 5, wherein the Fc region of human IgG1 has an amino acid sequence as shown in SEQ ID NO.3, and the Fc region variant of human IgG1 with ADCC effect knocked out has an amino acid sequence as shown in SEQ ID NO.4.

7. The bifunctional fusion protein of claim 4, wherein IL-15 and IL-15R Sushi domain are human or murine proteins; preferably,
the murine IL-15 has an amino acid sequence as shown in SEQ ID NO.7, and the murine IL-15R Sushi domain has an amino acid sequence as shown in SEQ ID NO.8;
the human IL-15 has an amino acid sequence as shown in SEQ ID NO.13, and the human IL-15R Sushi domain has an amino acid sequence as shown in SEQ ID NO.14.

8. A nucleotide fragment encoding the bifunctional fusion protein of any one of claims 1-7, or a vector or a host comprising the nucleotide fragment.

9. Use of the bifunctional fusion protein according to any one of claims 1-7, or the nucleotide fragment encoding the bifunctional fusion protein according to claim 8, or a vector or a host comprising the nucleotide fragment in the preparation of a medicament for:
(1) treating tumors; or
(2) treating tumors in combination therapy.

10. The use of claim 9, wherein
the tumor treatment is accomplished by reducing and removing intratumoral Treg cells and inducing proliferation of CD8+ T cells; preferably, the tumor is a tumor in an immunosuppressive state;
in the combination therapy, the medicament is a combined with the immune checkpoint inhibitor and may overcome immune checkpoint inhibitor tolerance; wherein the immune checkpoint inhibitor includes, but is not limited to: a PD-1/PD-L1 antibody, CTLA4 antibody, TIGIT antibody, LAG-3 antibody and TIM-3 antibody.
